# EUROPEAN PATENT SPECIFICATION

(11) **EP 2 646 800 B1**
(45) Date of publication and mention of the grant of the patent: **27.11.2019**
(21) Application number: 10800902.8
(22) Date of filing: 03.12.2010
(51) Int. Cl.: G01N 33/04, G01N 21/3577, G01N 21/05, G01N 21/35, G01N 21/85

(54) **MID-INFRARED SPECTRAL ANALYSIS OF A FLOWING HETEROGENEOUS MATERIAL**
MITTELINFRAROT-SPEKTRALANALYSE EINES HETEROGENEN STRÖMUNGSMATERIALS
ANALYSE SPECTRALE DE L'INFRAROUGE CENTRAL D'UN MATÉRIAU HÉTÉROGÈNE EN ÉCOULEMENT

(43) Date of publication of application: 09.10.2013
(73) Proprietor: FOSS Analytical A/S, 3400 Hillerød (DK)
(72) Inventor: JUHL, Henrik, DK-4000 Roskilde (DK)
(86) International application number: PCT/EP2010/068816
(87) International publication number: WO 2012/072143

(56) References cited:
- EP-A1- 0 393 459
- EP-A2- 1 715 327
- EP-A2- 1 715 327
- WO-A1-00/64242
- DE-A1- 10 352 924
- DE-A1- 10 352 924
- GB-A- 2 283 091
- GB-A- 2 283 091
- US-A- 4 247 773
- US-A- 4 247 773
- US-A1- 2004 135 088
- US-A1- 2004 135 088
- US-A1- 2004 179 194
- US-A1- 2010 285 523
- US-A1- 2010 285 523
- US-B1- 6 297 505
- US-B1- 6 297 505
- US-B2- 7 803 625

## Description

The present invention is defined in the claims and relates to a method of quantitatively determining components of a flowing heterogeneous material by mid-infrared spectral analysis (defined here as utilizing wavelengths from within the spectral region between 2.5µm - 10 µm), in particular to the determination of compositional parameters of a liquid in which particles are suspended, most particularly milk containing fat.

It is known to determine components of a sample , for example one or more of fat, lactose, glucose, protein, urea and/or adulterants in a fat-containing liquid samples, in particular blood, milk or milk product samples, or for example one or more of, protein, moisture and/or starch in cereal grains, by mid-infrared attenuation techniques. According to such techniques the sample is interrogated by transmitting in to the sample radiation in the mid-infrared spectral range. The attenuation of the interrogating mid-infrared radiation caused by the sample is then measured.

Systems, or instruments, for the measurement comprise mid-infrared attenuation measuring means for measuring the infrared attenuation of the sample in a number of wavebands, most usually across a continuous spectral range, and calculating means which are adapted to calculate the concentrations of components of interest in the sample based on the measured mid-infrared attenuation values of the sample. The calculation is performed using a calibration or predictive model by which is established a relationship between the component of interest and the measured mid-infrared attenuation values.

One problem associated with such a mid-infrared measurement on, for example, milk samples, is that the calculated results (often called indirect or predicted results, because the direct analysis results are results obtained using the standard chemical reference methods) vary with varying particle, here fat globule, size distribution in the samples.

This can be demonstrated theoretically as will be discussed. Assuming a cuvette having a diameter of 8mm being filled with raw milk containing 4% fat. A typical light path through the sample is 0.05mm for mid-infrared radiation. The useful (i.e. illuminated) volume of the cuvette can be estimated to be 2.5mm³. The fat content is 4% by weight and the density of milk is typically 0.93 g/ml this results in a volume of fat present in the cuvette of around 0.12mm³.

Fat globules in heterogeneous (unhomogenized) milk are of the order of between 4µm and 10µm in diameter. Assuming that the size distribution of these follows a Poisson distribution then the theoretical repeatability of fat determination in a cuvette can be calculated as shown in Table 1 where the last row represents the repeatability of fat in a cuvette filled with unhomogenized milk - calculated for different sizes of fat globules.

**Table 1**

| **Diameter(µm)** | **4** | **5** | **6** | **7** | **8** | **9** | **10** |
|---|---|---|---|---|---|---|---|
| **Volume (x10-8 mm3)** | 3.35 | 6.54 | 11.3 | 18 | 26.8 | 38.2 | 52.4 |
| **Number(x106)** | 3.226 | 1.652 | 0.956 | 0.602 | 0.403 | 0.283 | 0.206 |
| **R(rel)** | 0.056 | 0.078 | 0.102 | 0.129 | 0.157 | 0.188 | 0.220 |
| **R(abs)** | 0.002 | 0.003 | 0.004 | 0.005 | 0.006 | 0.008 | 0.009 |

As is reasonably expected the repeatability becomes worse as the diameter of the fat globule increases. However, these are much better than those observed for unhomogenized milk held stationary in a cuvette where absolute repeatabilities of up to 0.1 are typically obtained.

In order to mitigate this problem known instruments or systems used for the measurements are adapted to measure, in their measuring compartments, on homogenized samples and contain built-in homogenizers which are supposed to ensure that the various samples subjected to measurement have been subject to identical homogenization so that they have identical particle size distribution. It may be readily demonstrated experimentally that repeatabilites observed for homogenized milk held stationary in a cuvette are very close to the theoretical values presented above. In milk, for example, homogenizers are intended work to provide particle sizes of between 0.2µm and 2µm. However, the homogenizers of the systems of instruments are subject to mechanical wear, which means that their homogenizing efficiency decreases with time, thereby resulting in variations in the fat globule size distributions and thus to less accurate measurements.

One solution to avoid the need for homogenizers is provided in WO 92/17767. Here it is disclosed that mid-infrared attenuation measurements should be made on heterogeneous (unhomogenized) milk samples held stationary in a cuvette in the mid-infrared spectral region between 1160 cm -1 (8.62µm) and 1350 cm⁻¹ (7.41µm). This represents the region in which the C-O bond absorbs energy and was found to be unaffected by scatter from fat particles.

WO 2008/146276 describes a system which is adapted to make attenuation measurements on flowing, heterogeneous milk in the near-infrared spectral region and collecting measurement data both from light reflected by and light transmitted through the flowing milk. However, other components, for example water, have very strong influences on attenuation in this near-infrared region.

According to one aspect of the present invention there is provided a method of determining components of a flowing heterogeneous sample, comprising obtaining a sample of material; flowing the sample through a measurement region, such as may be provided by a flow through cuvette; concurrently interacting the flowing sample in the measurement region with mid-infrared radiation; subsequently measuring mid-infrared attenuation values at one or more wavebands typically from a spectrophotometric analysis of the interacted radiation in at least a wavelength region at which the component of interest influences the mid-infrared attenuation and calculating in a calculations means an indication of the component of interest in the sample from the measured mid-infrared attenuation values.

By making measurements on a flowing sample an effective averaging of the measurement may be conveniently produced and thereby an improved accuracy may be obtained. Measurements are repeated a plurality of times as the sample is flowed through the measurement region at a flow rate selected such that at least a portion of the sample in the measurement region is exchanged with new sample during the plurality of measurements, preferably with each measurement. Most preferably the flow rate is selected such that the entire sample in the measurement region is exchanged with each measurement.

It is generally accepted by a person skilled in the art that, contrary to near-infrared measurements, mid-infrared measurements on a flowing sample are to be avoided as poor accuracy and repeatability is expected, as will be explained below.

A particle in a suspension or a micelle in an emulsion typically contains different chemical bonds than the surrounding liquid, and each vibrational resonance of these bonds gives rise to a specific attenuation that, for example may be manifest as a specific frequency in an interferogram recorded by an attenuation measurement means of the interferometer type. If the particle or micelle is in a fixed position in the cuvette during the measurement time, the corresponding frequency and amplitude is constant across the unprocessed interferogram.

Typically, the interferogram is multiplied with a bell-shaped apodization function in order to smooth the discontinuities at the beginning and the end of the scan. Thus, if the particle or micelle is moving through the cuvette during the scan the resulting interferogram is affected. If the particle transits in the beginning or the end of the scan, the corresponding amplitude in the interferogram will be reduced due to the apodization. Consequently, after Fourier transformation of the apodized interferogram a particle that transits in the beginning or the end of the scan will have a smaller absorption peak than a particle that transits in the middle of the scan.

If the particle or micelle is moving very quickly across the cuvette during the measurement only a limited number of oscillations are recorded and the mid-infrared attenuation frequency (wavenumber) is not well-defined. This leads to a significant spreading of the absorption peak after the Fourier transform which also limits the measurement accuracy.

Naturally, with a large number of small particles or micelles in the flowing liquid, the recorded interferograms will represent an average and will be relatively unaffected by the flow. However, with an intermediate number of particles which are large compared to the volume of the illuminated cuvette - like fat globules in unhomogenized milk - the flow rate effects described above will influence the recorded interferogram and set the limit of the repeatability of the measurements.

The effect described here is more severe in the mid-infrared part of the spectrum using Fourier transform spectroscopy, than in the near-infrared part of the spectrum (typically considered to be wavelengths between 0.8µm and 2.5 µm). Firstly, since the absorption is much stronger in the mid-infrared range than in the near-infrared range, a much smaller volume of sample is measured in the mid-infrared range which makes the statistical fluctuations on the number of particles or micelles relatively larger. Secondly, since near-infrared measurements of flowing samples are usually performed with DDA (Diode Detector Array) spectrometers then the DDA will operate to average all spectral components (wavelengths) equally over time, eliminating the flow rate problem described above with respect to mid-infrared.

In one embodiment, when employed in the measurement of a fat containing liquid sample, such as milk or blood, the method may additionally include a step of warming the sample before interrogation with the mid-infrared radiation. This reduces the tendency of the suspended fat particles to agglomerate.

There is provided a mid-infrared attenuation measurement system for the quantitative determination of an indication of a component of interest in a heterogeneous flowable sample, the system comprising a flow conduit for insertion into a sample of the heterogeneous flowable material; transportation means coupled to the flow conduit to generate a flow of the sample therein; a mid-infrared attenuation measurement means adapted to supply mid-infrared radiation in to the sample as it is flowed and to generate a signal representative of a mid-infrared intensity variation of the supplied mid-infrared radiation after its passage through the flowing sample and a calculations means connected to receive the signal generated by the measurement means and to calculate the indication of the one or more components of interest depending on the received signal and on a predictive model, such as a provided by a calibration or by an artificial neural network, by which is established a mathematical relationship between mid-infrared attenuation values of the flowing heterogeneous material and the component of interest.

An exemplary embodiment of the present invention will now be described with reference to the drawings of the accompanying figures, of which:

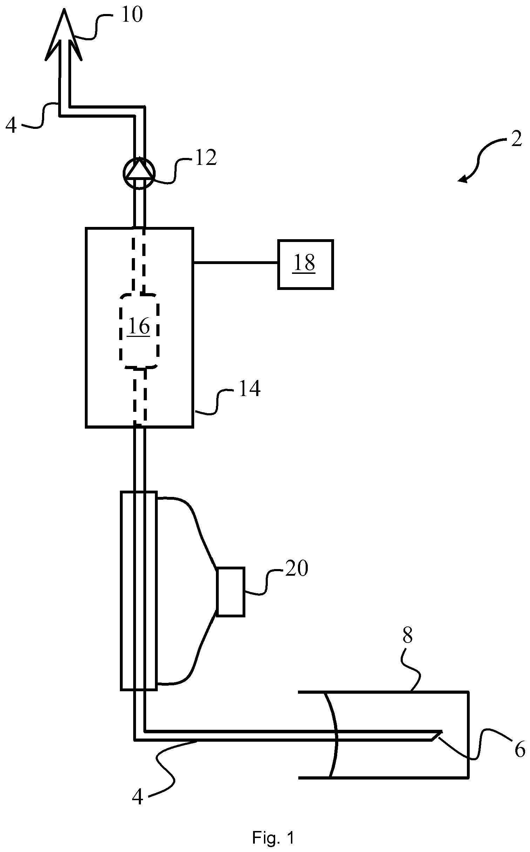

Fig. 1 illustrates a block diagram of an exemplary system operable to perform the method according to the present invention.

A mid-infrared attenuation measurement system 2 for the quantitative determination of an indication of a component of interest in a heterogeneous liquid sample is illustrated in Fig .1. The system 2 comprises a flow conduit 4 having a first end 6 for insertion into a heterogeneous liquid sample in a sample holder 8 and having a second end 10 for outputting the sample from the system 2, here connectable to waste. The system 2 also includes a transportation means 12, in the present example in the form of a pump, which is coupled to the flow conduit 8 and is operable to generate a flow through the conduit 4. A mid-infrared attenuation measurement means 14 is provided as a part of the system 2 for measuring attenuation of mid-infrared radiation which has interacted with the sample as it flows through a measurement region, here delimited by a flow through cuvette 16 which is provided in fluid connection with the sample flowing through the conduit 4.

A suitable mid-infrared attenuation measurement means 14 is an interferometer of the known type, for example a Michelson interferometer. This interferometer measurement means 14 is cooperatively disposed with respect to the measurement region 16, here defined by the flow through cuvette, to as to be able to detect mid-infrared radiation after transmission through the sample. In use the interferogram produced by the interferometer is processed using Fourier transformation in order to generate a wavelength dependent intensity variation representing the attenuation of the mid-infrared radiation by the sample.

Generally, the measurement region 16 can be any region at which in use it is intended that the flowing sample is interrogated by mid-infrared radiation. In this manner at least a portion of the sample being measured is exchanged during any measurement period. This then provides an effective average measurement which improves the accuracy and repeatability of the measurement results.

A calculations means 18, for example comprising an integral microprocessor or a stand-alone personal computer or a distributed system having at least one component at a location remote of the system 2 and operably coupled by a telecommunications network, is connected to receive a signal representative of the measured mid-infrared attenuation, such as an interferogram or the Fourier transformation of the same, and is configured to calculate in a known manner, an indication, such as a determined concentration, of a component of interest in the sample using a calibration or other predictive model (Artificial Neural Networks for example) by which is established a mathematical relationship between mid-infrared attenuation values and the component of interest.

A heater unit 20 may be included in specific embodiments for particular measurement applications in order to heat the sample before it is flowed through the measurement cuvette 16. The heater unit may for example comprise an electrically resistive coil wound around the conduit 4.

In a particular system 2 for the measurement of heterogeneous (unhomogenized) milk or milk product samples a heater is most usefully included in order to heat the milk sample to around 41°C. This reduces the tendency of fat particles in the milk to agglomerate. Heating may also be advantageously employed when measuring other fat containing liquids such as blood.

The results of determinations of typical components of interest, here represented as percentage fat, protein, lactose, Total Solids (TS) and Solids, Non Fat (SNF) in milk samples are provided in Table 2 together with absolute and relative accuracies A(abs) and A(rel) as well as absolute and relative repeatabilities R(abs) and R(rel) of these determinations.

**Table 2**

| **Component** | **Low** | **High** | **Mean** | **A(abs)** | **R(abs)** | **A(rel)** | **R(rel)** |
|---|---|---|---|---|---|---|---|
| Fat | 2.06 | 5.95 | 4.32 | 0.04 | 0.007 | 0.91 | 0.16 |
| Protein | 2.93 | 4.62 | 3.59 | 0.019 | 0.004 | 0.52 | 0.12 |
| Lactose | 4.33 | 5.48 | 4.67 | 0.032 | 0.005 | 0.69 | 0.11 |
| TS | 11.00 | 16.23 | 13.31 | 0.051 | 0.017 | 0.39 | 0.13 |
| SNF | 8.65 | 10.99 | 9.37 | 0.012 | 0.006 | 0.13 | 0.06 |

These determinations were made according to the method of the present invention using a system as described with respect to Fig.1.

Fifteen milk samples were used and measurements were made in three replicates for each sample with each replicate being an average of forty scans across the same wavelength region. In order to be able to construct a calibration model some of the samples had protein, fat and or lactose deliberately added in known amounts. The column Low represents the lowest amount of the respective component in a sample, **High** the highest amount and **Mean** the mean of all samples. By way of example only a partial Least Squares (PLS) calibration model using a maximum of six factors was then constructed in a known manner for use in subsequent predictions.

Each sample was interrogated by mid-infrared radiation and the resultant transmission interferograms processed by Fourier Transformation to a so-called "single beam" spectrum (i.e. an intensity dependent wavelength (or frequency) spectrum without corrections for external artefacts such as those induced by source; cuvette or detector). Transmittance was calculated relative to water in order to remove these artefacts not associated with interactions with the sample.

The samples were heated to 41°C and passed through the cuvette 16 at a flow rate of 1 ml/minute.

As can be seen the absolute accuracy A(abs) for all components is around 0.04 and the absolute repeatability R(abs) is around 0.01. This is surprising when considering that theoretically, as discussed above, mid-infrared measurements on a flowing sample are expected to be even worse than those made on a sample at standstill in a cuvette.

## Claims

1. A method of determining components of a flowing heterogeneous milk sample comprising obtaining a sample of heterogeneous milk; measuring mid-infrared attenuation values of the sample and calculating in a data processing unit an indication of the component of interest in the sample from the measured mid-infrared attenuation values **characterised in that** the method further comprises flowing the sample of heterogeneous milk ; concurrently interacting mid-infrared radiation with the flowing heterogeneous milk sample in a measurement region and subsequently measuring the mid-infrared attenuation values a plurality of times for a same one or more wavebands of the interacted radiation transmitted through the flowing heterogeneous milk sample as the sample of heterogeneous milk is flowed with a flow rate selected such that at least a portion of the sample of heterogeneous milk in the measurement region is exchanged for each of the plurality of times; and averaging the measured attenuation values.

2. A method according to Claim 1 **characterised in that** the method comprises a step of heating the sample of heterogeneous milk before measuring.

## Patentansprüche

1. Verfahren zum Bestimmen von Bestandteilen einer fließenden heterogenen Milchprobe, umfassend Gewinnen einer Probe von heterogener Milch; Messen von Abschwächungswerten der Probe im mittleren Infrarotbereich und Berechnen, in einer Datenverarbeitungseinheit, einer Indikation des interessierenden Bestandteils in der Probe aus den gemessenen Abschwächungswerten im mittleren Infrarotbereich, **dadurch gekennzeichnet, dass** das Verfahren weiter umfasst: Versetzen der Probe von heterogener Milch in Fluss; Bewirken einer übereinstimmenden Interaktion zwischen Strahlung im mittleren Infrarotbereich und der fließenden heterogenen Milchprobe in einem Messbereich und anschließendes Messen der Abschwächungswerte im mittleren Infrarotbereich mehrere Male für ein gleiches oder mehrere Wellenbänder der interagierenden Strahlung, die durch die fließende heterogene Milchprobe übertragen wird, während die Probe von heterogener Milch mit einer derart ausgewählten Flussrate in Fluss versetzt wird, dass zumindest ein Teil der Probe von heterogener Milch in dem Messbereich bei jedem der mehreren Male ausgetauscht wird; und Mitteln der gemessenen Abschwächungswerte.

2. Verfahren nach Anspruch 1, **dadurch gekennzeichnet, dass** das Verfahren einen Schritt eines Erwärmens der Probe von heterogener Milch vor dem Messen umfasst.

## Revendications

1. Procédé de détermination de composants d'un échantillon de lait hétérogène s'écoulant, comprenant obtenir un échantillon de lait hétérogène ; mesurer des valeurs d'atténuation d'infrarouge moyen de l'échantillon et calculer, dans une unité de traitement de données, une indication du composant d'intérêt dans l'échantillon à partir des valeurs d'atténuation d'infrarouge moyen, **caractérisé par le fait que** le procédé comprend en outre faire s'écouler l'échantillon de lait hétérogène ; faire interagir simultanément un rayonnement d'infrarouge moyen avec l'échantillon de lait hétérogène s'écoulant, dans une région de mesure, puis mesurer les valeurs d'atténuation d'infrarouge moyen une pluralité de fois pour une même ou plusieurs gammes d'ondes du rayonnement en interaction transmis à travers l'échantillon de lait hétérogène s'écoulant, à mesure que l'échantillon de lait hétérogène s'écoule avec un débit sélectionné de telle sorte qu'au moins une partie de l'échantillon de lait hétérogène dans la région de mesure est échangée pour chacune de la pluralité de fois ; et calculer une moyenne des valeurs d'atténuation mesurées.

2. Procédé selon la revendication 1, **caractérisé par le fait que** le procédé comprend une étape de chauffage de l'échantillon de lait hétérogène avant mesure.
